# EUROPEAN PATENT APPLICATION

(11) **EP 2 258 674 A1**
(43) Date of publication of application: **08.12.2010**
(21) Application number: 09006159.9
(22) Date of filing: 06.05.2009
(51) Int. Cl.: C07C 7/00, C07C 7/12, C07C 7/13, C07C 7/17, C07C 11/02

(54) **Method for removal of an organic amine from a hydrocarbon stream**

(71) Applicant: Saudi Basic Industries Corporation, 11422 Riyadh (SA); Linde AG, 80331 München (DE)
(72) Inventor: Mosa, Fuad, 11551 Riyadh (SA); Azam, Shahid, 11551 Riyadh (SA); Ghouse Rafi, Mohammed, 11422 Riyadh (SA); Ali, Syed, 11551 Riyadh (SA); Al-Onizi, Abdullah, 11422 Riyadh (SA); Fritz, Peter, M., Dr., 82008 Unterhaching (DE); Bölt, Heinz, 85515 Wolfratshausen (DE); Meiswinkel, Andreas, Dr., 81479 München (DE); Taube, Carsten, Dr., 85560 Ebersberg (DE); Winkler, Florian, 80469 München (DE); Müller, Wolfgang, Dr., 81245 München (DE); Wöhl, Anina, 82049 Pullach (DE); Göke, Volker, Dr., 82515 Wolfratshausen (DE); Schneider, Richard, 82449 Uffing (DE); Fritz, Helmut, 81373 München (DE)
(74) Representative: Scholz, Volker

(57) **Abstract**

The present invention relates to a method for removal of an organic amine from a hydrocarbon stream containing amine, comprising the steps of: reacting the amine of the hydrocarbon stream containing the amine with an acid, optionally extracting the amine-salt formed into an aqueous phase, and optionally recovering and recycling of the amine. The present invention also relates to a method for removal of an organic amine from a hydrocarbon stream containing the amine, comprising the steps: passing the hydrocarbon stream containing the amine to an ion exchange resin and loading the amine onto that resin, and optionally recovering and recycling of the amine. Moreover, the present invention relates to a method for removal of an organic amine from a hydrocarbon stream containing the amine, comprising the steps: passing the hydrocarbon stream containing the amine through an adsorbing agent selected from the group of inorganic compounds, such as silica gel, alumina and molecular sieve, and optionally recovering and recycling of the amine.

## Description

The present invention relates to a method for removal of an organic amine from a hydrocarbon stream.

In the chemical industry, processes are often conducted resulting in an outlet stream product or a feed stream to a process unit comprising hydrocarbons and amines. An example thereof is the outlet stream from a reactor utilized for preparing linear alpha-olefins by oligomerization of ethylene. The linear alpha-olefins produced are then separated into different fractions for further use or marketing. Often, an amine is added during the oligomerization process or is added into a reactor outlet piping system. Such processes are, for example, disclosed in US-A-5811619 or the non-published PCT/EP2009/000030. In other processes amines are utilized as corrosion inhibitors or for adjustment of the pH value.

In many cases, it is difficult to remove the organic amine from the hydrocarbon stream by distillation as the boiling points of the amine and the hydrocarbon stream (or fractions thereof) are very close. For example, n-dodecylamine (DDA) is often added in an oligomerization, which after the product fractionation finally ends up in the C₁₄-LAO-product fraction. Since DDA has a boiling point close to the C₁₄-product, it cannot be removed by distillation.

Presently, no commercially available method is known for the removal of an amine from a respective hydrocarbon stream. The comparably high concentration of the amine in the product stream complicates its removal.

As a consequence, in this case the LAO-product with a certain concentration of the amine has to be marketed under consideration of its amine content, which may restrict its applicability for certain downstream processes. For example, the amine could represent a poison in processes with sensitive catalytic reactions.

It is therefore an object of the present invention to provide a method for removal of an organic amine from a hydrocarbon stream which overcomes the drawbacks of the prior art. Especially, a method shall be provided enabling that the hydrocarbon product can be marketed without any restriction due to its amine content. Further, a method shall be preferably provided wherein the amine can be recovered and recycled.

In a first embodiment, the object is achieved by a method for removal of an organic amine from a hydrocarbon stream containing the amine, comprising the steps:
- reacting the amine of the hydrocarbon stream containing the amine with an acid,
- optionally extracting the amine-salt formed into an aqueous phase, and
- optionally recovering and recycling of the amine.

In a second embodiment, this object is achieved by a method for removal of an organic amine from a hydrocarbon stream containing the amine, comprising the steps:
- passing the hydrocarbon stream containing the amine to an ion exchange resin and loading the amine onto that resin, and
- optionally recovering and recycling of the amine.

In a third embodiment, the object is achieved by a method for removal of an organic amine from a hydrocarbon stream containing the amine, comprising the steps:
- passing the hydrocarbon stream containing the amine through an adsorbing agent selected from the group of inorganic compounds such as silica gel, alumina and molecular sieve, and
- optionally recovering and recycling of the amine.

Preferably, the hydrocarbon stream containing the amine is an outlet stream from a reactor for preparing linear alpha-olefins (LAO) by oligomerization of ethylene or a fraction of such an outlet stream.

Even preferred the fraction is a product fraction in the range of C₁₀ to C₁₈ , and/or the amine may be n-decylamine, tributylamine, 2-ethylhexylamine, n-dodecylamine or trihexylamine.

The first embodiment preferably comprises recovering the amine by reacting the amine-salt with caustic, separating the resulting amine and recycling it.

As an acid any commercially available organic or inorganic acid can be used in concentrated or diluted form. Preferably, the acid is aqueous HCl or sulfuric acid. More preferably, the acid is aqueous HCl in a concentration of 10 wt%, preferably in a concentration of 5 wt%, or sulfuric acid.

The second embodiment preferably comprises recovering the amine by regenerating the ion exchange resin with acid and/or caustic, preferably in a polar solvent and separating the amine obtained and recycling it.

The third embodiment preferably comprises recovering the amine by regenerating the adsorbing agent and separating the amine obtained and recycling it.

Regeneration is preferably achieved in the third embodiment by exposure of the adsorbing agent to temperatures of at least 100 °C or flushing the adsorbing agent with a polar solvent such as water, toluene, formic acid or alcohols.

For the regeneration of the adsorbent also any suitable dry gas can be utilized, like fuel gas, nitrogen from the plant nitrogen header, the methane fraction or the hydrogen fraction (tail gas) from a steam cracker.

Preferably, guard beds are provided for the purified hydrocarbon stream and the recovered amine for removal of trace components.

In a further preferred embodiment, the recovered amine is dried by distillation.

If the amine is recovered by reacting the amine-salt with caustic according to the first embodiment, a carrier medium may be preferably utilized for improvement of the separation of the amine from the aqueous phase.

Finally, the reactor for preparing linear-alpha olefins (LAO) by oligomerization of ethylene may contain a catalyst comprising a zirconium component and an organoaluminum component, preferably a zirconium component having the formula ZrCl₄₋ₘXₘ, wherein X = OCOR or OSO₃R' with R and R' being independently alkyl, alkene and phenyl, and wherein 0 ≤ m ≤ 4, and wherein the organoaluminum compound being preferably Al(C₂H₅)₃, Al₂Cl₃(C₂H₅)₃, AlCl(C₂H₅)₂ or a mixture thereof.

Surprisingly, it was found that the inventive method for removing an organic amine from a hydrocarbon stream provides finally a hydrocarbon product which can be marketed without any restriction due to its amine content. Further, the inventive method allows easy and actually complete removal of the amine from the hydrocarbon stream. Additionally, the cost for the amine utilized in respective chemical reaction processes can be considerably reduced, since the amine can be recovered and recycled.

This is especially true for a method for preparing linear alpha-olefins, as disclosed above, wherein an organic amine is added into the oligomerization reaction and/or into the reactor outlet piping system. It was calculated that cost savings in an amount of several million euro per year for a typical commercial plant for the oligomerization of ethylene can be achieved.

In a most preferred embodiment of the invention, the method for removal of an organic amine from a hydrocarbon stream containing the amine according to the invention is embedded in a method for preparing linear alpha-olefins (LAO) by oligomerization of ethylene, preferably in the presence of solvent and catalyst, comprising the steps:
(i) feeding ethylene into an oligomerization reactor,
(ii) oligomerizing the ethylene in the reactor,
(iii) removing a reactor outlet stream comprising linear alpha-olefins from the reactor via a reactor outlet piping system,
(iv) optionally transferring the reactor outlet stream to a catalyst deactivation and removal step, and
(v) optionally deactivating and removing the catalyst from the reactor outlet stream,
wherein at least one organic amine is added into the oligomerization reactor and/or into the reactor outlet piping system. The reactor outlet stream or a fraction thereof can then be taken as the hydrocarbon stream in the present invention.

Additional features and advantages of the inventive method will now become apparent from the detailed description of embodiments thereof.

In all embodiments illustrated below, the hydrocarbon stream is the product fraction in the range of C₁₀ to C₁₈ , containing an amine obtained from a method for preparing linear alpha-olefins, as for example disclosed in PCT/EP2009/000030.

According to the first embodiment, the amine in the amine containing hydrocarbon stream is reacted with an acid, for example aqueous HCl, according to the following chemical equation:

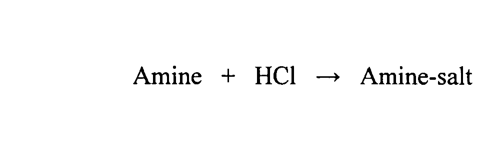

The amine salt is soluble in an aqueous phase and, as a consequence, can then be extracted from the hydrocarbon phase into an aqueous phase.

In a further processing step, the amine can be recovered, and it can be even reused for dosing into the LAO reactor outlet lines or the oligomerization reactor, e.g. by realizing the following chemical equation:

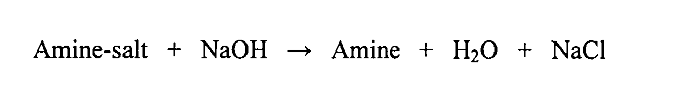

In this reaction step the caustic (NaOH) can also be replaced by other media like Ca(OH)₂.

In the reaction steps of this embodiment, mixing elements, like static or dynamic mixers, may be utilized for optimizing the reaction efficiency. Further, after the treatment with acid and caustic and the separation of the amine form the hydrocarbon phase, the amine obtained can be washed with water several times to minimize the amount of entrained acid/caustic.

The different steps (reaction with acid, caustic or washing with water) can be performed in a once-through mode, or the reaction/washing efficiencies can be enhanced by installation of cycles. In addition, the separation efficiencies can be improved by introduction of a suitable carrier stream. The carrier may be an external hydrocarbon stream or the recovered amine. Additionally, for the hydrocarbon stream and the recycled amine adequate guard beds or distillations can be installed in accordance with required specifications (for example removal of moisture, trace components, salts, etc.).

In the second embodiment of the present invention the amine of the amine containing hydrocarbon stream can be removed by means of an ion exchange resin. For that reasons, the amine containing hydrocarbon stream is passed to a suitable exchange resin for loading the amine on that resin. A suitable ion exchange resin may be selected from the group of strong acidified resins, weak acidified resins or highly crosslinked resins. After a certain operation period, the ion exchange resin has to be regenerated which can be achieved by flushing the resin with acid and/or caustic.

After the regeneration step, the amine can be also recovered and used for recycling into the method for oligomerization of ethylene.

The third embodiment of the present invention provides a method for removal of an organic amine from a hydrocarbon stream containing the amine, wherein the amine containing hydrocarbon stream is passed through an adsorbing material selected from silica gel, alumina and molecular sieve. Here, the amine is adsorbed on the adsorbing material. After a certain operation period, the adsorbing material has to be regenerated. This can be achieved by, for example, thermal regeneration, i.e. exposure of the adsorbing material to higher temperatures in a range of 100 to 200 °C , or by solvent regeneration, i.e. flushing the adsorbing material with a suitable solvent.

The amine obtained after regeneration can be also recovered and recycled for the corresponding purpose.

In a preferred embodiment of the present invention, the methods for removal of an organic amine might be also combined, i.e. the amine of the hydrocarbon stream containing the amine might be both passed to an ion exchange resin and passed through an adsorbing agent. For example, the amine might be removed from an outlet stream of an oligomerization reactor as follows:

In a first step, the amine is extracted from the outlet stream, typically the C₁₀ - C₁₈ LAO-product stream, by extraction of the amine by a polar solvent, such as methanol, for example using a simple centrifuge which can achieve a removal of the amine of about 90%. Then, the upper phase from the extraction which contains the LAO and about 10% of the remaining amine can be treated by an ion exchange resin. The bottom phase which contains the methanol with the extracted amine can be fed to a separation column where methanol will be recycled back for extraction again, and the amine will be sent back to the main process (oligomerization).

The advantages of this scheme are a reduction of process equipment, reduced waste generation and utilization of conventional materials for most of the utilized equipment.

The features disclosed in the foregoing description and in the claims may, both separately and in any combination thereof, be material for realizing the invention in diverse forms therof.

## Claims

1. Method for removal of an organic amine from a hydrocarbon stream containing the amine, comprising the steps of:
- reacting the amine of the hydrocarbon stream containing the amine with an acid,
- optionally extracting the amine-salt formed into an aqueous phase, and
- optionally recovering and recycling of the amine.

2. Method for removal of an organic amine from a hydrocarbon stream containing the amine, comprising the steps:
- passing the hydrocarbon stream containing the amine to an ion exchange resin and loading the amine onto that resin, and
- optionally recovering and recycling of the amine.

3. Method for removal of an organic amine from a hydrocarbon stream containing the amine, comprising the steps:
- passing the hydrocarbon stream containing the amine through an adsorbing agent selected from the group of inorganic compounds, such as silica gel, alumina and molecular sieve, and
- optionally recovering and recycling of the amine.

4. Method according to any of the claims 1-3, wherein the hydrocarbon stream containing the amine is an outlet stream from a reactor for preparing linear alpha-olefins (LAO) by oligomerization of ethylene or a fraction of such an outlet stream.

5. Method according to claim 4, wherein the fraction is the product fraction in the range of C₁₀ to C₁₈.

6. Method according to claim 1, comprising recovering the amine by reacting the amine-salt with caustic, separating the resulting amine and recycling it.

7. Method according to claim 1, wherein the acid is aqueous HCl in a concentration of 10 wt %, preferably in a concentration of 5 wt%, or sulfuric acid.

8. Method according to claim 2, comprising recovering the amine by regenerating the ion exchange resin with acid and/or caustic, preferably in a polar solvent, and separating the amine obtained and recycling it.

9. Method according to claim 3, comprising recovering the amine by regenerating the adsorbing agent and separating the amine obtained and recycling it.

10. Method according to claim 9, wherein regenerating is achieved by exposure of the adsorbing agent to temperatures of at least 100 °C or flushing the adsorbing agent with a polar solvent.

11. Method according to any of the preceding claims, wherein guard beds are provided for the purified hydrocarbon stream and the recovered amine for removal of trace components.

12. Method according to any of the preceding claims, wherein the recovered amine is dried by distillation.

13. Method according to claim 6, wherein a carrier medium is utilized for improvement of the separation of the amine from the aqueous phase.

## Amended claims

### Amended claims in accordance with Rule 137(2) EPC.

**1.** Method for removal of an organic amine from a hydrocarbon stream containing the amine, comprising the steps of:
- reacting the amine of the hydrocarbon stream containing the amine with an acid and forming an amine salt, and
- extracting the amine-salt formed into an aqueous phase,
wherein the hydrocarbon stream containing the amine is an outlet stream from a reactor for preparing linear alpha-olefins (LAO) by oligomerization of ethylene or a fraction of such an outlet stream, wherein the reactor contains a catalyst comprising a zirconium component and an organoaluminum component.

**2.** Method according to claim 1, comprising the additional step of recovering and recycling of the amine after extraction.

**3.** Method according to claim 1 or 2, wherein the fraction is the product fraction in the range of C₁₀ to C₁₈.

**4.** Method according to claim 2, comprising recovering the amine by reacting the amine-salt with caustic, separating the resulting amine and recycling it.

**5.** Method according to claim 1, wherein the acid is aqueous HCl in a concentration of 10 wt %, preferably in a concentration of 5 wt%, or sulfuric acid.

**6.** Method according to any of the preceding claims 2 to 5, wherein the recovered amine is dried by distillation.
